# EUROPEAN PATENT APPLICATION

(11) **EP 3 922 183 A1**
(43) Date of publication of application: **15.12.2021**
(21) Application number: 20178732.2
(22) Date of filing: 08.06.2020
(51) Int. Cl.: A61B 6/06, A61B 6/00

(54) **APPARATUS FOR CORRECTION OF COLLIMATOR PENUMBRA IN AN X-RAY IMAGE**

(71) Applicant: Koninklijke Philips N.V., 5656 AG Eindhoven (NL)
(72) Inventor: KOEHLER, Thomas, 5656 AE Eindhoven (NL); MAACK, Hanns-Ingo, 5656 AE Eindhoven (NL)
(74) Representative: Philips Intellectual Property & Standards

(57) **Abstract**

The present invention relates to an apparatus (10) for correction of collimator penumbra in an X-ray image. The apparatus comprises an input unit (20), a processing unit (30), and an output unit (40). The input unit is configured to provide the processing unit with X-ray data. The processing unit is configured to determine at least one collimator corrected X-ray image of an object. The determination comprises application of an intensity modulation mask to the X-ray data. The intensity modulation mask accounts for intensity variation across a detector of an X-ray acquisition system caused by at least one collimator blade of the X-ray acquisition system, and the X-ray acquisition system was used to acquire the X-ray data. The output unit is configured to output the at least one collimator corrected X-ray image of the object.

## Description

### FIELD OF THE INVENTION

The present invention relates to an apparatus for correction of collimator penumbra in an X-ray image, to an x-ray imaging system, to a method for correction of collimator penumbra in an X-ray image, as well as to a computer program element and a computer readable medium.

### BACKGROUND OF THE INVENTION

X-ray imaging systems collimate the X-ray beam to limit exposure of the patient. Collimator blades are used to closely match the exposed field of view to the extent of the detector. In order to ensure that the patient is exposed as little as possible with x-rays that are not detected, the collimator blades are positioned such that, ideally, the x-ray intensity falls to zero at the edge of the detector or slightly within the detector area, or exceptionally slightly outside of the detector area. Ideally the collimator edges should never be outside of the detector area but the in many cases they can be located within the detector area, when for example the patient requires a collimation smaller than the detector. However, because the source of x-rays is not infinitely small, a penumbra effect caused by the collimator blades is observed, where the radiation intensity gradually falls from 100% to 0%.

Such X-ray beam collimation is conducted in standard radiography attenuation (or transmission) x-ray imaging systems, and also in interferometry based differential phase contrast and dark field imaging systems. Such phase contrast and dark field imaging systems provide a dark field image, phase contrast image, and an attenuation (transmission) image.

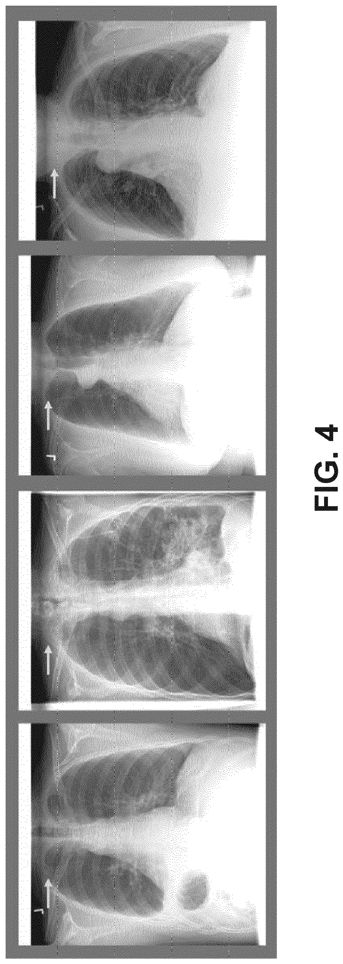

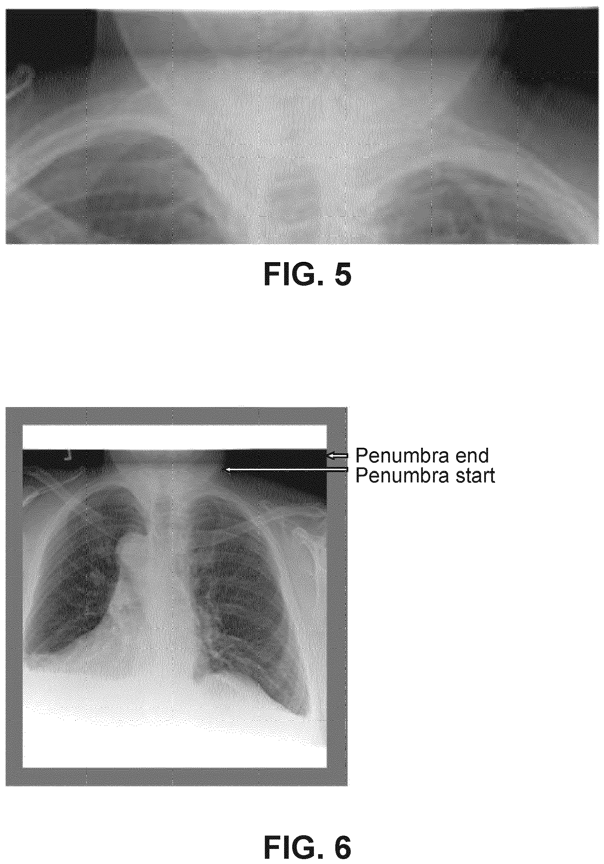

Fig. 4 shows illustrations of horizontal artifacts caused by the collimator blades in x-ray transmission images. During a clinical study, these horizontal artifacts were observed in particular in the transmission images. A detailed analysis shows that the artifact is caused by the blurred penumbra of the collimator blade. In the geometry at hand, the penumbra extends a few tens of the pixels. Consequently, there is an additional intensity gradient in the acquired raw data that comes on top of the intensity variation due to the patient. Often in the neck or shoulder region, the x-ray intensity increases from bottom to top, whereas the intensity gradient due to the collimator blur decreases. The combination of the two effects leads in typical cases to a pronounced intensity maximum, which is perceived as a horizontal artifact, clearly visible in Fig. 4. Fig. 5 also shows an expanded x-ray transmission image where the horizontal artifact caused by collimator penumbra is visible.

Fig. 6 shows more detail of this problem, where the penumbra is illustrated on an example case where the transmission is shown with a display window ranging from 0 to 1. The penumbra is clearly visible, and it is to be noted that the penumbra is much broader at the top than the bottom due to the shallow anode angle.

Although expert clinicians can observe the penumbra effect in many situations, it can lead to difficulties in interpretation of imagery, and this is an increased issue for more inexperienced staff. Also, with the move towards automated analysis of imagery, such collimator induced artifacts can lead to spurious or incorrect image interpretation.

There is a need to address this issue.

### SUMMARY OF THE INVENTION

It would be advantageous to improve X-ray imaging, where collimator blades are utilized to limit the field of view of the X-ray beam with respect to the detector area.

The object of the present invention is solved with the subject matter of the independent claims, wherein further embodiments are incorporated in the dependent claims. It should be noted that the following described aspects and examples of the invention apply also to the apparatus for correction of collimator penumbra in an X-ray image, to the x-ray imaging system, to the method for correction of collimator penumbra in an X-ray image, as well as for the computer program element and computer readable medium.

According to a first aspect, there is provided an apparatus for correction of collimator penumbra in an X-ray image. The apparatus comprises:
- an input unit;
- a processing unit; and
- an output unit.

The input unit is configured to provide the processing unit with X-ray data. The processing unit is configured to determine at least one collimator corrected X-ray image of an object, wherein the determination comprises application of an intensity modulation mask to the X-ray data. The intensity modulation mask accounts for intensity variation across a detector of an X-ray acquisition system caused by at least one collimator blade of the X-ray acquisition system, and the X-ray acquisition system was used to acquire the X-ray data. The output unit is configured to output the at least one collimator corrected X-ray image of the obj ect.

In this manner intensity gradients resulting from collimator penumbra can be corrected for in radiography attenuation x-ray images, and the intensity gradients can be corrected in attenuation x-ray images resulting from a dark field and phase contrast interferometric X-ray imaging system. The effect of such intensity gradients in the dark field and phase contrast images themselves can also be mitigated.

In an example, the X-ray data comprises an X-ray attenuation image of the object, and the X-ray acquisition system was an attenuation image acquisition system. The application of the intensity modulation mask comprises a multiplication of the intensity values in the X-ray attenuation image of the object associated with pixels of the detector by corresponding intensity values in the intensity modulation mask, and wherein the at least one collimator corrected X-ray image of the object comprises a collimator corrected attenuation X-ray image.

Thus, over the majority of the image detector values have not been reduced and can be considered to be "unity", but due to the effect of the collimator blades, normally at the extremes of the image, the intensity values gradually fall from such a unity value towards 0. Thus, by determining this intensity profile a mask can be determined that is the inverse of this, having a value 1.0 over most of the mask where the image data was "unity", but at the extremes of the mask rising from 1.0 to high levels over the part of the mask corresponding to parts of the image affected by the collimator penumbra. This image data can then be multiplied by this mask to correct for the penumbra effect. Clearly, rather than taking an inverse of the intensity values, intensity values themselves could be utilised as a mask and this mask used as a dividing mask rather than a multiplying mask.

In other words, although the penumbra is not an actual feature of the object, the collimator blades can lead to a penumbra effect leading to intensity gradients at the edge of the attenuation image of the object that can lead to problems in relation to interpretation or utilization of the attenuation image. For example, as described in US2015/342554A1, an attenuation image can be used for scatter correction for Compton scatter. Within this procedure, the attenuation image data is turned into equivalent water thickness. For this "water object", there is a model of Compton scatter and the penumbra effect leads to an overestimation of water and so to an overestimation of Compton scatter. Also, for dark field imaging there is a known correction of the image based on the transmission or attenuation image. This corrects the influence of beam hardening, which reduces the dark field as well. The penumbra will also lead to an overestimation of this effect.

Thus, in addition to the penumbra effect leading to artifacts in the attenuation image itself, that can lead to image interpretation difficulties, the attenuation image itself when used in augmenting other image modalities can cause issues. However, the new technique described here addresses these issues by correcting for the effect of the penumbra.

In an example, the X-ray acquisition system was an attenuation image acquisition system, or an interferometric image acquisition system.

In an example, the processing unit is configured to determine the intensity modulation mask, the determination comprising utilization of the X-ray attenuation image of the object.

In an example, the X-ray data comprises an X-ray attenuation image with no object present, and the X-ray acquisition system was an attenuation image acquisition system or an interferometric image acquisition system. The processing unit is configured to determine the intensity modulation mask. The determination can then comprise utilization of the X-ray attenuation image with no object present.

In an example, the determination of the intensity modulation mask comprises an identification of at least one intensity gradient in the X-ray attenuation image of the object or in the X-ray attenuation image with no object present, and where the at least one intensity gradient is associated with the at least collimator blade.

In an example, the processing unit is configured to annotate the collimator corrected attenuation X-ray image with at least one location of the at least one gradient associated with the at least one collimator blade.

Thus, with the medical professional views the resultant x-ray imagery they can see where the data have been corrected to mitigate the effect of the collimator penumbra, thus with respect to the correction, the medical professional can see where this correction has been applied and can use their professional to better interpret the underlying feature knowing that a correction has been applied.

In an example, when the X-ray acquisition system was an interferometric image acquisition system and the at least one collimator corrected X-ray image of the object comprises a collimator corrected dark field X-ray image and/or a collimator corrected phase contrast X-ray image. The processing unit is configured to annotate the collimator corrected dark field X-ray image and/or the collimator corrected phase contrast X-ray image with at least one location of the at least one gradient associated with the at least one collimator blade.

In an example, determination of the intensity modulation mask comprises utilization of a machine learning algorithm implemented by the processing unit.

In an example, the machine learning algorithm comprises at least one trained neural network.

In this way, an automatic system can be used to determine the intensity modulation mask. Humans can determine where the collimator penumbra have affected imagery, and by training a neural network with a series of training imagery with ground truth information from a medical expert identifying where the penumbra is to be found, the system can determine where a penumbra is located even for a system using a movable x-ray source, where the resultant collimator penumbra also move.

In an example, the X-ray data comprises blank scan fringe data and object scan fringe data, wherein the X-ray acquisition system was an interferometric image acquisition system. The application of the intensity modulation mask comprises a multiplication of the intensity values in the X-ray blank scan fringe data and X-ray object scan fringe data associated with pixels of the detector by corresponding intensity values in the intensity modulation mask to determine pre-processed X-ray blank scan fringe data and pre-processed X-ray object scan fringe data. The processing unit is configured to determine a dark field image of the object and/or a phase contrast image of the object comprising application of a phase retrieval algorithm to the pre-processed blank scan fringe data and to the pre-processed object scan fringe data. The at least one collimator corrected X-ray image of the object then comprises a collimator corrected dark field X-ray image and/or a collimator corrected phase contrast X-ray image.

Even though the effect of the collimator penumbra are most pronounced in a radiography attenuation x-ray image, and in an attenuation x-ray image that can also be acquired along with the dark field and phase contrast image of a interferometric DAX system, the dark field and phase contrast image data can also be corrected for the penumbra effect.

According to a second aspect, there is provided an X-ray imaging system. The system comprises:
- an X-ray acquisition system; and
- an apparatus for correction of collimator penumbra in an X-ray image according to the first aspect.

In an example, the X-ray acquisition system is an attenuation image acquisition system, or an interferometric image acquisition system.

According to a third aspect, there is provided a method for correction of collimator penumbra in an X-ray image, the method comprising:
a) providing a processing unit with X-ray data;
c) determining by the processing unit at least one collimator corrected X-ray image of an object, wherein the determining comprises applying an intensity modulation mask to the X-ray data, wherein the intensity modulation mask accounts for intensity variation across a detector of an X-ray acquisition system caused by at least one collimator blade of the X-ray acquisition system, and wherein the X-ray acquisition system was used to acquire the X-ray data; and
f) outputting by an output unit the at least one collimator corrected X-ray image of the object.

According to another aspect, there is provided a computer program element controlling apparatus as previously described which, if the computer program element is executed by a processing unit, is adapted to perform the method steps as previously described.

According to another aspect, there is provided a computer readable medium having stored computer element as previously described.

The computer program element, can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of an apparatus for correction of collimator penumbra in an X-ray image;
Fig. 2 shows a schematic set up of an example of an x-ray imaging system;
Fig. 3 shows a method for correction of collimator penumbra in an X-ray image;
Figs. 4 and 5 illustrate artifacts caused by collimator penumbra in a number of X-ray transmission images;
Fig. 6 illustrates the extent of the collimator induced artifacts in a transmission X-ray image;
Fig. 7 illustrates the operation of the apparatus, system and method of Figs. 1-3, where the left top image shows a transmission X-ray image with a collimator penumbra induced artifact extending across the top of the image, the centre top image shows an intensity modulation mask used to correct the left image, and the right top image shows the corrected image where the intensity in the left image has been divided by the intensities in the mask, and where the bottom images show enlarged views of the top images; and
Fig. 8 shows a schematic set up of an example of a phase contrast and/or dark field imaging system.

### DETAILED DESCRIPTION OF EMBODIMENTS

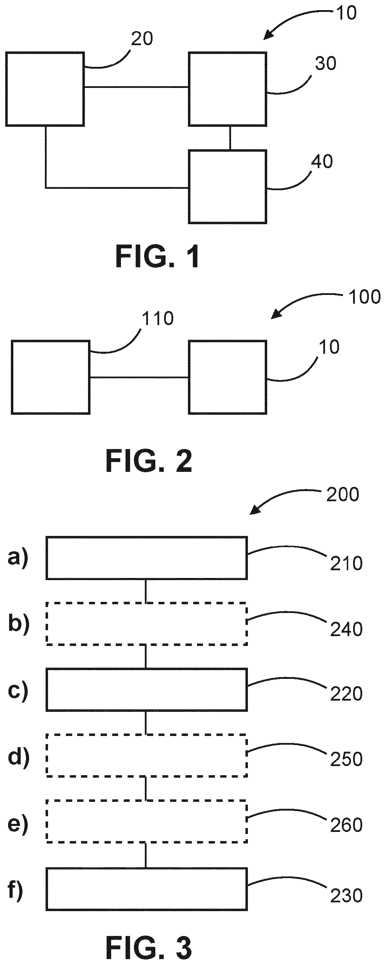

Fig. 1 shows a schematic example of an apparatus 10 for correction of collimator penumbra in an X-ray image. The apparatus 10 comprises an input unit 20, a processing unit 30, and an output unit 40. The input unit is configured to provide the processing unit with X-ray data. The processing unit is configured to determine at least one collimator corrected X-ray image of an object. The determination of the at least one collimator corrected X-ray image of the object comprises application of an intensity modulation mask to the X-ray data. The intensity modulation mask accounts for intensity variation across a detector of an X-ray acquisition system caused by at least one collimator blade of the X-ray acquisition system, and the X-ray acquisition system was used to acquire the X-ray data. The output unit is configured to output the at least one collimator corrected X-ray image of the object.

According to an example, the X-ray data comprises an X-ray attenuation image of the object, and the X-ray acquisition system was an attenuation image acquisition system. The application of the intensity modulation mask can comprise a multiplication of the intensity values in the X-ray attenuation image of the object associated with pixels of the detector by corresponding intensity values in the intensity modulation mask, and the at least one collimator corrected X-ray image of the object comprises a collimator corrected attenuation X-ray image.

It is to be noted that "attenuation image" is here, and elsewhere referred to as the raw data image. Thus, it is the raw data image that is corrected for the penumbra effect, and this corrected image can then have the logarithm of the data carried out to provide an image that would be the image normally viewed by the clinician.

In an example, the intensity modulation mask is in effect an inverse of the above intensity modulation mask. Thus in this example, the X-ray data comprises an X-ray attenuation image of the object, and the X-ray acquisition system was an attenuation image acquisition system. The application of the intensity modulation mask comprises a division of the intensity values in the X-ray attenuation image of the object associated with pixels of the detector by corresponding intensity values in the intensity modulation mask, and the at least one collimator corrected X-ray image of the object comprises a collimator corrected attenuation X-ray image.

According to an example, the X-ray acquisition system was an attenuation image acquisition system, or an interferometric image acquisition system.

According to an example, the processing unit is configured to determine the intensity modulation mask. The determination can comprise utilization of the X-ray attenuation image of the object.

According to an example, the X-ray data comprises an X-ray attenuation image with no object present, and the X-ray acquisition system was an attenuation image acquisition system, or the X-ray acquisition system was an interferometric image acquisition system. The processing unit is configured to determine the intensity modulation mask. The determination can comprise utilization of the X-ray attenuation image with no object present.

According to an example, the determination of the intensity modulation mask comprises an identification of at least one intensity gradient in the X-ray attenuation image of the object or in the X-ray attenuation image with no object present, and where the at least one intensity gradient is associated with the at least collimator blade.

According to an example, the processing unit is configured to annotate the collimator corrected attenuation X-ray image with at least one location of the at least one gradient associated with the at least one collimator blade.

According to an example, when the X-ray acquisition system was an interferometric image acquisition system the at least one collimator corrected X-ray image of the object then comprises a collimator corrected dark field X-ray image and/or a collimator corrected phase contrast X-ray image. The processing unit is configured to annotate the collimator corrected dark field X-ray image and/or the collimator corrected phase contrast X-ray image with at least one location of the at least one gradient associated with the at least one collimator blade.

According to an example, determination of the intensity modulation mask comprises utilization of a machine learning algorithm implemented by the processing unit.

According to an example, the machine learning algorithm comprises at least one trained neural network.

According to an example, the X-ray data comprises blank scan fringe data and object scan fringe data, wherein the X-ray acquisition system was an interferometric image acquisition system. The application of the intensity modulation mask can then comprise a multiplication of the intensity values in the X-ray blank scan fringe data and X-ray object scan fringe data associated with pixels of the detector by corresponding intensity values in the intensity modulation mask to determine pre-processed X-ray blank scan fringe data and pre-processed X-ray object scan fringe data. The processing unit is configured to determine a dark field image of the object and/or a phase contrast image of the object comprising application of a phase retrieval algorithm to the pre-processed blank scan fringe data and to the pre-processed object scan fringe data. The at least one collimator corrected X-ray image of the object then comprises a collimator corrected dark field X-ray image and/or a collimator corrected phase contrast X-ray image.

In an example, the intensity modulation mask is in effect an inverse of the above intensity modulation mask. Thus in this example, the X-ray data comprises blank scan fringe data and object scan fringe data, wherein the X-ray acquisition system was an interferometric image acquisition system. The application of the intensity modulation mask can then comprise a division of the intensity values in the X-ray blank scan fringe data and X-ray object scan fringe data associated with pixels of the detector by corresponding intensity values in the intensity modulation mask to determine pre-processed X-ray blank scan fringe data and pre-processed X-ray object scan fringe data. The processing unit is configured to determine a dark field image of the object and/or a phase contrast image of the object comprising application of a phase retrieval algorithm to the pre-processed blank scan fringe data and to the pre-processed object scan fringe data. The at least one collimator corrected X-ray image of the object then comprises a collimator corrected dark field X-ray image and/or a collimator corrected phase contrast X-ray image.

Fig. 2 shows a schematic example of an X-ray imaging system 100. The system 100 comprises an X-ray acquisition system 110, and an apparatus 10 for correction of collimator penumbra in an X-ray image as described with respect to Fig. 1.

According to an example, the X-ray acquisition system is an attenuation image acquisition system, or the X-ray acquisition system is an interferometric image acquisition system.

Fig. 3 shows a method 200 for correction of collimator penumbra in an X-ray image in its basic steps, where essential steps are shown in bold lines and optional steps are shown in dashed lines. The method 200 comprises:
- in a providing step 210, also referred to as step a), providing a processing unit with X-ray data;
- in a determining step 220, also referred to as step c), determining by the processing unit at least one collimator corrected X-ray image of an object, wherein the determining comprises applying an intensity modulation mask to the X-ray data, wherein the intensity modulation mask accounts for intensity variation across a detector of an X-ray acquisition system caused by at least one collimator blade of the X-ray acquisition system, and wherein the X-ray acquisition system was used to acquire the X-ray data; and
- in an outputting step 230, also referred to as step f), outputting by an output unit the at least one collimator corrected X-ray image of the object.

In an example, the X-ray data comprises an X-ray attenuation image of the object, wherein the X-ray acquisition system was an attenuation image acquisition system, and wherein in step c) applying the intensity modulation mask comprises multiplying the intensity values in the X-ray attenuation image of the object associated with pixels of the detector by corresponding intensity values in the intensity modulation mask, and wherein the at least one collimator corrected X-ray image of the object comprises a collimator corrected attenuation X-ray image.

In an example, the X-ray acquisition system was an attenuation image acquisition system, or an interferometric image acquisition system.

In an example, the method comprises step b) determining 240 by the processing unit the intensity modulation mask, the determining comprising utilizing the X-ray attenuation image of the object.

In an example, the X-ray data comprises an X-ray attenuation image with no object present, wherein the X-ray acquisition system was an attenuation image acquisition system, or an interferometric image acquisition system, and wherein step b) comprises utilizing the X-ray attenuation image with no object present.

In an example, step b) comprises identifying at least one intensity gradient in the X-ray attenuation image of the object or in the X-ray attenuation image with no object present, wherein the at least one intensity gradient is associated with the at least collimator blade.

In an example, the method comprises step d) annotating 250 by the processing unit the collimator corrected attenuation X-ray image with at least one location of the at least one gradient associated with the at least one collimator blade.

In an example, when the X-ray acquisition system was an interferometric image acquisition system the at least one collimator corrected X-ray image of the object comprises a collimator corrected dark field X-ray image and/or a collimator corrected phase contrast X-ray image, and wherein the method comprises step e) annotating 260 by the processing unit the collimator corrected dark field X-ray image and/or the collimator corrected phase contrast X-ray image with at least one location of the at least one gradient associated with the at least one collimator blade.

In an example, step b) comprises utilizing a machine learning algorithm implemented by the processing unit.

In an example, the machine learning algorithm comprises at least one trained neural network.

In an example, the X-ray data comprises blank scan fringe data and object scan fringe data, wherein the X-ray acquisition system was an interferometric image acquisition system, and wherein in step c) applying the intensity modulation mask comprises multiplying the intensity values in the X-ray blank scan fringe data and X-ray object scan fringe data associated with pixels of the detector by corresponding intensity values in the intensity modulation mask to determine pre-processed X-ray blank scan fringe data and pre-processed X-ray object scan fringe data, wherein step c) comprises determining by the processing unit a dark field image of the object and/or a phase contrast image of the object comprising applying a phase retrieval algorithm to the pre-processed blank scan fringe data and to the pre-processed object scan fringe data; and wherein the at least one collimator corrected X-ray image of the object comprises a collimator corrected dark field X-ray image and/or a collimator corrected phase contrast X-ray image.

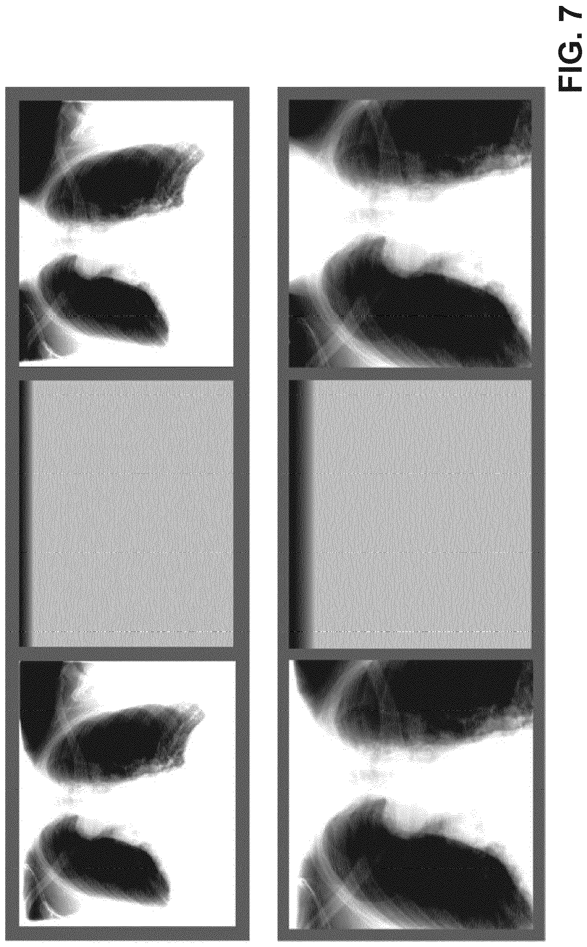

The apparatus and method for correction of collimator penumbra in an X-ray image, and the X-ray imaging system are now described with respect to specific embodiments, where reference is made to Figs. 7-8.

Fig. 7 shows on the left a transmission image with the collimator induced artefact extending across the image at the top, below which is an expanded part of the image. In effect, a number of vertical cross sections through the image data would be modulated by intensity variations caused by the patient's body, and then near to the top of the image and intensity gradient is imposed upon the data due to the collimator penumbra, where the intensity gradually falls. This information can be used to determine an intensity modulation mask, that accounts for the collimator penumbra. Thus the image itself can be used to determine the intensity modulation mask. The intensity modulation mask, can then be in effect the intensity variation across detector without the patient, and therefore can have a value 1.0 almost everywhere, but at the top of the mask corresponding the top of the image fall from 1.0 towards 0. Such a mask is shown in the centre top image of Fig. 7, again with an expanded version shown below this image. This mask can then be used as a dividing mask to divide the intensities by the mask values to determine a corrected x-ray transmission image, as shown in the right hand image of Fig. 7, again shown at the top as the whole image and below that in an expanded form. The mask values can be the inverse of the values discussed above, and be 1.0 almost everywhere, but rise from 1.0 to high values across the collimator penumbra area. This mask can then be used as the multiplying mask, in the same way as a dividing mask discussed above.

Rather than use image data with a patient present, a null transmission image with no patient object present can be taken, and the intensity modulation mask determined. Here, the advantages that no intensity modulation due to the patient has to be taken into account.

Intensity modulation masks can be determined for different focal spot sizes and positions, and indeed sourced image distances if this is variable in a system. These intensity modulation masks can be predetermined for different system settings, but also can be determined "on-the-fly" for imagery as it is required.

The determination of the intensity modulation masks can also involve application of an intensity modulation mask to imagery as discussed above, and then a variation of the intensity modulation mask until the effect of the collimator penumbra is minimised.

Also, a trained machine learning algorithm such as a neural network can be utilised to determine the intensity modulation mask. A human can visually see the collimator penumbra induced artifacts, and the machine learning algorithm can be trained on a number of training imagery is with associated ground truth data of the position of the artifacts, thereby enabling it to identify penumbra in newly acquired imagery and determine the required intensity modulation mask.

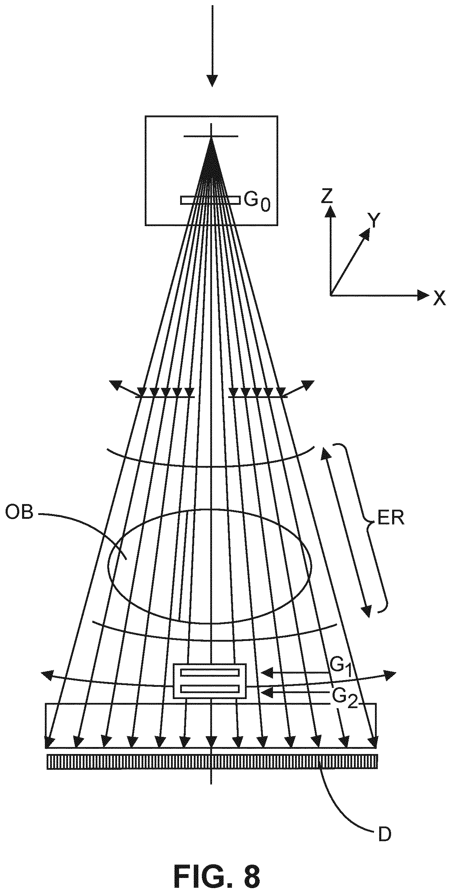

The intensity modulation mask can be applied to transmission or attenuation x-ray imagery acquired by a standard radiography system. However, the intensity modulation mask can also be applied in a dark field and phase contrast interferometry based x-ray imaging system. Such a system is discussed below with respect to Fig. 8, but in essence movement of grating within a grating arrangement with and without an object is conducted. Active movement of the grating leads to a sinusoid or modulation of pixel values across detector, with a phase retrieval process enabling visibility information to derive a dark field image, phase information to derive a phase contrast image, and with mean values being used to determine an absorption, or transmission, or attenuation image. The above processing with the intensity modulation mask can be applied to the attenuation image in the same way as discussed above for a standard radiography attenuation image. However, for the correction of dark field and phase contrast imagery, the scan images themselves can be corrected by the intensity modulation mask prior to a phase retrieval step that produces the dark field and phase contrast images themselves. It is however to be noted that the collimator penumbra induced artifacts in dark field and phase contrast imagery is not as noticeable as for the attenuation imagery. In addition to application of the intensity modulation mask to the scan data itself, because the effective focal spot size decreases in the penumbra region, this leads to increased visibility of the fringes and this effect can be modelled to further to correct for the collimator penumbra induced artifacts in dark field and phase contrast imagery.

Standard attenuation x-ray imaging systems are commonplace, however dark field or phase contrast interferometer based x-ray imaging systems constitute a newly developing field of x-ray imagery. As such, for completeness this new imaging technology is briefly discussed below with reference to Fig. 8.

For the acquisition of the dark field and phase contrast data, as well as the attenuation data, a two (Talbot type) or three-grating (Talbot-Lau type) interferometer is introduced into the X-ray beam, normally termed G0, G1 and G2 gratings. An exemplar system is shown in Fig. 8, where typically G0 and G2 are absorber gratings and G1 is a phase grating, and where an object OB is placed within an examination region ER. The source grating G0, can be used to make radiation from the source more coherent but is not always necessary, and gratings G1 and G2 are normally termed phase and analyzer gratings. Subsequently, for a so-called full field system, one of the two gratings G1 or G2 is moved perpendicular to the grating lamellae relative to the other gratings in a number of steps (so-called stepping), and if the source grating G0 is utilized it can be this grating that is stepped laterally (where laterally means perpendicular to the grating direction). Thereby, for each new grating position an image is recorded on the detector D. Comparison of the image sequence acquired with and without a sample in the beam, allows to calculate the three imaging signals: transmission or attenuation (conventional X-ray image), phase contrast image, and dark field image. These gratings generate a fringe pattern on top of the conventional transmission image, and for example the dark field signal is calculated as the loss of contrast of this fringe pattern. The fringe-pattern, which is analyzed in dark field and phase contrast imaging, is a fine structure in the micrometer range. Using an analyzer grating with the same periodicity, a Moire-pattern can be measured with the detector. Any movement of one or more interferometer components, such as the analyzer grating, in this length scale changes the phase of the Moire-pattern. Instead of using a full-field system and phase stepping, a scanning type of system can be used as described e.g. in US 9959640 B2.

Thus, a sample or object, the body in Fig. 8, modulates attenuation, refraction, and small angle scattering information onto the radiation. To separate phase information from other contributions to the signal, such as attenuation by the sample, inhomogeneous illumination or imperfections of the gratings, a phase "stepping" approach is utilized. One of the gratings (either G1 or G2 - or G0 if present) is scanned along the transverse direction over at least one period of the grating, and for every point of the scan an image is taken (it is to be noted that the intensity modulation mask is applied to this scan). The resultant phase contrast, dark field, and attenuation data then oscillates sinusoidal, with and without the sample, and this can be utilized to determine the dark field, phase contrast and attenuation images using a phase retrieval algorithm. Further detail on the standard phase stepping approach can be found in the paper by Weitkamp et al, Optics Express, Vol. 13, No. 16, (2005) 6296-6304.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.
This exemplary embodiment of the invention covers both, a computer program that right from the beginning uses the invention and computer program that by means of an update turns an existing program into a program that uses invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. Apparatus (10) for correction of collimator penumbra in an X-ray image, the apparatus comprising:
- an input unit (20);
- a processing unit (30); and
- an output unit (40);
wherein, the input unit is configured to provide the processing unit with X-ray data;
wherein, the processing unit is configured to determine at least one collimator corrected X-ray image of an object, wherein the determination comprises application of an intensity modulation mask to the X-ray data, wherein the intensity modulation mask accounts for intensity variation across a detector of an X-ray acquisition system caused by at least one collimator blade of the X-ray acquisition system, and wherein the X-ray acquisition system was used to acquire the X-ray data; and
wherein, the output unit is configured to output the at least one collimator corrected X-ray image of the object.

2. Apparatus according to claim 1, wherein the X-ray acquisition system was an attenuation image acquisition system, or an interferometric image acquisition system.

3. Apparatus according to claim 1-2, wherein the X-ray data comprises an X-ray attenuation image of the object, wherein the X-ray acquisition system was an attenuation image acquisition system, and wherein application of the intensity modulation mask comprises a multiplication of the intensity values in the X-ray attenuation image of the object associated with pixels of the detector by corresponding intensity values in the intensity modulation mask, and wherein the at least one collimator corrected X-ray image of the object comprises a collimator corrected attenuation X-ray image.

4. Apparatus according to any of claims 2-3, wherein the processing unit is configured to determine the intensity modulation mask, the determination comprising utilization of the X-ray attenuation image of the object.

5. Apparatus according to any of claims 1-4, wherein the X-ray data comprises an X-ray attenuation image with no object present, wherein the X-ray acquisition system was an attenuation image acquisition system, or an interferometric image acquisition system, and wherein the processing unit is configured to determine the intensity modulation mask, the determination comprising utilization of the X-ray attenuation image with no object present.

6. Apparatus according to any of claims 4-5, wherein the determination of the intensity modulation mask comprises an identification of at least one intensity gradient in the X-ray attenuation image of the object or in the X-ray attenuation image with no object present, wherein the at least one intensity gradient is associated with the at least collimator blade.

7. Apparatus according to claim 6, wherein the processing unit is configured to annotate the collimator corrected attenuation X-ray image with at least one location of the at least one gradient associated with the at least one collimator blade.

8. Apparatus according any of claims 6-7, wherein when the X-ray acquisition system was an interferometric image acquisition system the at least one collimator corrected X-ray image of the object comprises a collimator corrected dark field X-ray image and/or a collimator corrected phase contrast X-ray image and wherein the processing unit is configured to annotate the collimator corrected dark field X-ray image and/or the collimator corrected phase contrast X-ray image with at least one location of the at least one gradient associated with the at least one collimator blade.

9. Apparatus according to any of claims 4-8, wherein determination of the intensity modulation mask comprises utilization of a machine learning algorithm implemented by the processing unit.

10. Apparatus according to claim 9, wherein the machine learning algorithm comprises at least one trained neural network.

11. Apparatus according to claim 1, wherein the X-ray data comprises blank scan fringe data and object scan fringe data, wherein the X-ray acquisition system was an interferometric image acquisition system, and wherein application of the intensity modulation mask comprises a multiplication of the intensity values in the X-ray blank scan fringe data and X-ray object scan fringe data associated with pixels of the detector by corresponding intensity values in the intensity modulation mask to determine pre-processed X-ray blank scan fringe data and pre-processed X-ray object scan fringe data, wherein the processing unit is configured to determine a dark field image of the object and/or a phase contrast image of the object comprising application of a phase retrieval algorithm to the pre-processed blank scan fringe data and to the pre-processed object scan fringe data; and wherein the at least one collimator corrected X-ray image of the object comprises a collimator corrected dark field X-ray image and/or a collimator corrected phase contrast X-ray image.

12. An X-ray imaging system (100), the system comprising:
- an X-ray acquisition system (110); and
- an apparatus (10) for correction of collimator penumbra in an X-ray image according to any of claims 1-11.

13. System according to claim 12, wherein the X-ray acquisition system is an attenuation image acquisition system, or an interferometric image acquisition system.

14. A method (200) for correction of collimator penumbra in an X-ray image, the method comprising:
a) providing (210) a processing unit with X-ray data;
c) determining (220) by the processing unit at least one collimator corrected X-ray image of an object, wherein the determining comprises applying an intensity modulation mask to the X-ray data, wherein the intensity modulation mask accounts for intensity variation across a detector of an X-ray acquisition system caused by at least one collimator blade of the X-ray acquisition system, and wherein the X-ray acquisition system was used to acquire the X-ray data; and
f) outputting (230) by an output unit the at least one collimator corrected X-ray image of the object.

15. A computer program element for controlling an apparatus according to one of claims 1 to 11 and/or system of any of claims 12-13, which when executed by a processor is configured to carry out the method of claim 14.
